# EUROPEAN PATENT SPECIFICATION

(11) **EP 1 428 875 B1**
(45) Date of publication and mention of the grant of the patent: **28.06.2006**
(21) Application number: 02293085.3
(22) Date of filing: 13.12.2002
(51) Int. Cl.: C12N 9/88, A01K 67/027, A61K 48/00

(54) **Method of diagnosis of obesity**
Verfahren zur Diagnose von Fettsucht
Procédé de diagnostic de l'obésité

(43) Date of publication of application: 16.06.2004
(73) Proprietor: CENTRE NATIONAL DE LA RECHERCHE SCIENTIFIQUE (CNRS), 75016 Paris (FR)
(72) Inventor: Boutin, Philippe, 59200 Tourcoing (FR); Dubois, Séverine, 80800 Vecquemont (FR); Dina, Christian, 75009 Paris (FR); Froguel, Philippe, 93170 Bagnolet (FR)
(74) Representative: Warcoin, Jacques

(56) References cited:
- WO-A-00/25798
- WO-A-98/37224
- WO-A-02/102143
- JOHNSON GILLIAN C L ET AL: "A comprehensive, statistically powered analysis of GAD2 in type 1 diabetes." DIABETES, vol. 51, no. 9, September 2002 (2002-09), pages 2866-2870, XP002246819 September, 2002 ISSN: 0012-1797
- PRICE R A ET AL: "A locus affecting obesity in human chromosome region 10p12." DIABETOLOGIA, vol. 44, no. 3, March 2001 (2001-03), pages 363-366, XP002246820 ISSN: 0012-186X
- BU D-F ET AL: "TWO HUMAN GLUTAMATE DECARBOXYLASES, 65-KDA GAD AND 67-KDA GAD, ARE EACH ENCODED BY A SINGLE GENE" PROCEEDINGS OF THE NATIONAL ACADEMY OF SCIENCES OF USA, NATIONAL ACADEMY OF SCIENCE. WASHINGTON, US, vol. 89, no. 6, 15 March 1992 (1992-03-15), pages 2115-2119, XP000608332 ISSN: 0027-8424
- DATABASE BIOSIS [Online] BIOSCIENCES INFORMATION SERVICE, PHILADELPHIA, PA, US; 20 November 2002 (2002-11-20) JUN H S ET AL: "Role of glutamic acid decarboxylase in the pathogenesis of type 1 diabetes." Database accession no. PREV200300107150 XP002246821 & CMLS CELLULAR AND MOLECULAR LIFE SCIENCES, vol. 59, no. 11, 20 November 2002 (2002-11-20), pages 1892-1901, ISSN: 1420-682X
- GENG L ET AL: "WIDESPREAD EXPRESSION OF AN AUTOANTIGEN-GAD65 TRANSGENE DOES NOT TOLERIZE NON-OBESE DIABETIC MICE AND CAN EXACERBATE DISEASE" PROCEEDINGS OF THE NATIONAL ACADEMY OF SCIENCES OF USA, NATIONAL ACADEMY OF SCIENCE. WASHINGTON, US, vol. 95, August 1998 (1998-08), pages 10055-10060, XP002942662 ISSN: 0027-8424
- FAGIOLINI M.; HENSCH T.: 'inhibitory threshold for critical period activation in primary visual cortex' NATURE vol. 404, March 2000, pages 183 - 186

### SEQUENCE LISTING

<110> CENTRE NATIONAL DE LA RECHERCHE SCIENTIFIQUE (CNRS)
<120> METHOD OF DIAGNOSIS OF OBESITY
<130> 64 808 - EP
<140>
   <141>
<160> 15
<170> Patentln Ver. 2.1
<210> 1
   <211> 1758
   <212> DNA
   <213> Homo sapiens
<220>
   <223> gad2 gene
<400> 1
   atggcatctc cgggctctgg cttttggtct ttcgggtcgg aagatggctc tggggattcc 60
   gagaatcccg gcacagcgcg agcctggtgc caagtggctc agaagttcac gggcggcatc 120
   ggaaacaaac tgtgcgccct gctctacgga gacgccgaga agccggcgga gagcggcggg 180
   agccaacccc cgcgggccgc cgcccggaag gccgcctgcg cctgcgacca gaagccctgc 240
   agctgctcca aagtggatgt caactacgcg tttctccatg caacagacct gctgccggcg 300
   tgtgatggag aaaggcccac tttggcgttt ctgcaagatg ttatgaacat tttacttcag 360
   tatgtggtga aaagtttcga tagatcaacc aaagtgattg atttccatta tcctaatgag 420
   cttctccaag aatataattg ggaattggca gaccaaccac aaaatttgga ggaaattttg 480
   atgcattgcc aaacaactct aaaatatgca attaaaacag ggcatcctag atacttcaat 540
   caactttcta ctggtttgga tatggttgga ttagcagcag actggctgac atcaacagca 600
   aatactaaca tgttcaccta tgaaattgct ccagtatttg tgcttttgga atatgtcaca 660
   ctaaagaaaa tgagagaaat cattggctgg ccagggggct ctggcgatgg gatattttct 720
   cccggtggcg ccatatctaa catgtatgcc atgatgatcg cacgctttaa gatgttccca 780
   gaagtcaagg agaaaggaat ggctgctctt cccaggctca ttgccttcac gtctgaacat 840
   agtcattttt ctctcaagaa gggagctgca gccttaggga ttggaacaga cagcgtgatt 900
   ctgattaaat gtgatgagag agggaaaatg attccatctg atcttgaaag aaggattctt 960
   gaagccaaac agaaagggtt tgttcctttc ctcgtgagtg ccacagctgg aaccaccgtg 1020
   tacggagcat ttgaccccct cttagctgtc gctgacattt gcaaaaagta taagatctgg 1080
   atgcatgtgg atgcagcttg gggtggggga ttactgatgt cccgaaaaca caagtggaaa 1140
   ctgagtggcg tggagagggc caactctgtg acgtggaatc cacacaagat gatgggagtc 1200
   cctttgcagt gctctgctct cctggttaga gaagagggat tgatgcagaa ttgcaaccaa 1260
   atgcatgcct cctacctctt tcagcaagat aaacattatg acctgtccta tgacactgga 1320
   gacaaggcct tacagtgcgg acgccacgtt gatgttttta aactatggct gatgtggagg 1380
   gcaaagggga ctaccgggtt tgaagcgcat gttgataaat gtttggagtt ggcagagtat 1440
   ttatacaaca tcataaaaaa ccgagaagga tatgagatgg tgtttgatgg gaagcctcag 1500
   cacacaaatg tctgcttctg gtacattcct ccaagcttgc gtactctgga agacaatgaa 1560
   gagagaatga gtcgcctctc gaaggtggct ccagtgatta aagccagaat gatggagtat 1620
   ggaaccacaa tggtcagcta ccaacccttg ggagacaagg tcaatttctt ccgcatggtc 1680
   atctcaaacc cagcggcaac tcaccaagac attgacttcc tgattgaaga aatagaacgc 1740
   cttggacaag atttataa 1758
<210> 2
   <211> 2382
   <212> DNA
   <213> Homo sapiens
<220>
   <223> 5' flanking region of gad2 gene nucleotides
<220>
   <223> R = G or A
<220>
   <223> Y = T or C
<220>
   <223> M = A or C
<220>
   <223> W = A or T
<220>
   <223> S = G or C
<400> 2
   gaaaaaaaaa tcagttaaat tcaggtgttt taatccgttt cttctttggg ggttttgtgt 60
   gatttaaacg cttgctttta agaaccttta tgttttcaac cactcatcca tagtagaaaa 120
   gttctgcaac cctagactgc tggcttgaag gaaaaccttt gcaggatttg atatggattt 180
   caacaaagaa ccagcctctg cgaggctgga gagagctgcg gagctgccat gcctgaagtg 240
   cagatggctg aaccacaagt ctttaggttt ccggagttgt tattgtggtg acctagagtg 300
   tcagagccag gagagcaaga aagaggagcc aaactgagcc ctgagttttc gaccacccgg 360
   gctcccacag cctggwacag acttcaccta gcacgctcag tgccagcctt cggcaggacg 420
   ctatcaacgc ccgactggwt tcytgctctc atcctggcgc ctgggcccag ttgccatagt 480
   gtggatccca tgactcctca gggaacccct ggactcaggc acgcgagaag aagacagcgc 540
   tttgtggaga gaattgacca gggacagtta tgctcgagca cacaggactt gggcctgtat 600
   gcgtccagca tgggccccag gatgtccctt ctaagcgagg gtcgaggggt gctcgcccag 660
   acgggatccc cgggtctctg ctttgttagc agctttggtg gctggttcag gaggtcagag 720
   aaataaaacg acttgtgaac acaatggaaa tgacaggcgc tctggccagg cgcggggaar 780
   gcagccgcct cgggaagccg acctcagccc tttcctctct ctccctccct ccgtctcccc 840
   cagagccccg gagctccgag gtgcacttga agttcatctc cactgccagg agaacgcagc 900
   gcaaactgtc aaagggctcc caatccttag ggcgtcctcc ctgtctgcaa tagctttttg 960
   tagaaaggaa atcatcagaa mgattctgac tttctcctcc tttctttatt agaaaaagag 1020
   aaaccgcctc ctaatacgcc tcagagagaa ccaatctcgc gcttccgggt cacccgctac 1080
   cgcgaagatt ctctgggggc gagggggggc attggtttga agccccttaa aacgagggcc 1140
   ctgcaggcga tgccttcttt cctactcgga tttgtaaagc cgagattgct tagttggaaa 1200
   ccctgttctc ccctcccagg cgcacacaga tcccccttac acgcaagcag cgggcgcttc 1260
   cacgcctccg cgggccaagg tcaccaaatg ccctgattcc atcccccacc cgccatcaat 1320
   cctgccgact ctggccgctc tgcctcattc tcttccaaga agtttccatt cgttttattt 1380
   ttttttcccc agcccgaggt cctcagtaga ctccagcgtg gattttaatt gcctcaatca 1440
   gcagtcattc tccccagccg tcactcagag cctggacggt gggtcccgcg atctagccct 1500
   tggctacgca ggaacggtgc gcccccgggt acggcgcggt tcagcaggca ggcgtcaggt 1560
   tctaccaagg cgctgaaatg agcccatcag cgggtaggag cccttccccc gccgtcccct 1620
   ccccaggctc gtgaacggcg cctgatgccc gcccggggcg cgagctctcg aggtcgcagt 1680
   gacctcagca cctgcttgga ggaaaacggc gcgggaaccc cgcttccttc ccctcagctg 1740
   gagccagacc tcaaacaaaa ccccaatcga tgcacacaga aaactcctct gggccacgct 1800
   tcccgcctcg ccgaggtctc cccagtctgc ccctcgctga cgctggcgcg cagcsgctgt 1860
   ggcagcaccc gggacagcgg ccgcccgcac ttcccgcctc tggctcgccc gaggacgcgc 1920
   tggcacgcct cccaccccct cactctgact ccagctggcg tgcatggtct gcctcgcatc 1980
   ctcacgactc agctccctcc ctctctcgtg tttttttcct ccgccgcccc ctcattcatc 2040
   cccactgggc tccctttccc tcaaatgctc tggggctctc cgcgctttcc tgagtccggg 2100
   ctccgaggac ccttaggtag tcccggtctc ttttaargct ccccggcttc caaagggttg 2160
   ccacgtccct aaaccctgtc tccagctcgc atacacacac gcacagacac gcacgttttc 2220
   tgttcctgcg tgacacccgc cctcgccgct cggccccgcc ggtccccgcg cggtgccctc 2280
   ctcccgccac acgggcacgc acgcgcgcgc agggccaagc ccgaggcagc tcgcccgcag 2340
   ctcgcactcg caggcgacct gctccagtct ccaaagccga tg 2382
<210> 3
   <211> 519
   <212> PRT
   <213> Homo sapiens
<220>
   <223> DNA-binding protein Ikaros (Lymphoid transcription factor LyF-1)
<400> 3
<210> 4
   <211> 20
   <212> DNA
   <213> Artificial Sequence
<220>
   <223> Description of Artificial Sequence: Primer to amplify SNP - 243
<400> 4
cctcaaatgc tctggggctc 20
<210> 5
   <211> 20
   <212> DNA
   <213> Artificial Sequence
<220>
   <223> Description of Artificial Sequence: Primer to amplify SNP - 243
<900> 5
ggtgtcacgc aggaacagaa 20
<210> 6
   <211> 23
   <212> DNA
   <213> Artificial Sequence
<220>
   <223> Description of Artificial Sequence: Primer to amplify SNP - 243
<400> 6
gtctctttta aagctccccg gct 23
<210> 7
   <211> 28
   <212> DNA
   <213> Artificial Sequence
<220>
   <223> Description of Artificial Sequence: Primer to amplify SNP - 243
<400> 7
cgggctccga ggacccttag gtagtccc 28
<210> 8
   <211> 17
   <212> DNA
   <213> Artificial Sequence
<220>
   <223> Description of Artificial Sequence: Primer to amplify SNP - 1.6 kb
<400> 8
ctgaggcgta ttaggag 17
<210> 9
   <211> 17
   <212> DNA
   <213> Artificial Sequence
<220>
   <223> Description of Artificial Sequence: Primer to amplify SNP - 1.6 kb
<400> 9
ctcctaatac gcctcag 17
<210> 10
   <211> 16
   <212> DNA
   <213> Artificial Sequence
<220>
   <223> Description of Artificial Sequence: Primer to amplify SNP - 1.6 kb
<400> 10
ggaaagcagc cgcctc 16
<210> 11
   <211> 30
   <212> DNA
   <213> Artificial Sequence
<220>
   <223> Description of Artificial Sequence: Primer to amplify SNP - 1.6 kb
<400> 11
tggaaatgac aggcgctctg gccaggcgcg 30
<210> 12
   <211> 21
   <212> DNA
   <213> Artificial Sequence
<220>
   <223> Description of Artificial Sequence: Primer to amplify SNP - 2004
<400> 12
tgttttcaac cactcatcca t 21
<210> 13
   <211> 17
   <212> DNA
   <213> Artificial Sequence
<220>
   <223> Description of Artificial Sequence: Primer to amplify SNP - 2004
<400> 13
agggacagtt atgctcg 17
<210> 14
   <211> 18
   <212> DNA
   <213> Artificial Sequence
<220>
   <223> Description of Artificial Sequence: Primer to amplify SNP - 2004
<400> 14
acagcctggt acagactt 18
<210> 15
   <211> 23
   <212> DNA
   <213> Artificial Sequence
<220>
   <223> Description of Artificial Sequence: Primer to amplify SNP - 2004
<400> 15
tgagttttcg accacccggg ctc 23

## Description

The invention is in the field of human genetics and relates to new methods of diagnosis and therapy of obesity, in particular morbid obesity, based on the identification of polymorphisms in the 5' region of the *gad*2 gene.

Morbid obesity is a serious disease process, in which the accumulation of fatty tissue on the body becomes excessive, interferes with, or injures the other bodily organs, and creates (or predictably will create) serious and life-threatening health problems, which are called co-morbidities.

It is said that people are morbidly obese when their Body Mass Index (BMI = height (cm) / (mass (kg))²) is equal to or over 40.

Numerous scientific studies have established that there is a genetic predisposition to morbid obesity.

A region of the chromosome 10 has been involved in obesity (Hager et al, Nature Genetics 1998; 20:304-38.), which has been recently confirmed in a cohort of German young obese subjects (Hinney et al. 2000, Journal of Clinical Endocrinology and Metabolism 2000 ; 85 (8): 2962-5), as well as in White Caucasians and in African Americans (Price et al. 2001, Diabetologia 1999, 42 : 555-9), and in the old order Amish (Hsueh et al. 2001, The Journal of Clinical Endocrinology and Metabolism 2001 ; 86(3) : 1199-1205).

Three new single nucleotide polymorphisms (SNPs) have been identified in the frame of the invention, located in this region, and more particularly in the 5' region of the *gad*2 gene. These SNPs show positive association with obesity in morbidly obese subjects, while other SNPS in this region do not show this association.

In the frame of the present invention, by *gad*2 gene is meant to indicate a gene whose coding sequence is represented by SEQ ID N° 1.

The 5' flanking region of *gad*2 gene is meant to indicate the region comprising nucleotides 1-2379 of SEQ ID N° 2, and especially the region consisting in nucleotides 1-2379 of SEQ ID N° 2. Nucleotides 2380-2382 of SEQ ID N° 2 represent the start codon of the *gad*2 gene (ATG).

Thus, in a first embodiment, the invention relates to a method for diagnosing a predisposition for obesity, and in particular morbid obesity, in a human subject which comprises determining whether there is a germline alteration in the sequence of the 5' flanking region of the *gad*2 gene, wherein said alteration is the presence of at least one of the following mutations: -243 A>G at nucleotide 2137 of SEQ ID N° 2, -1.6kb G>A at nucleotide 780 of SEQ ID N° 2, -2004 A>T at nucleotide 376 of SEQ ID N° 2, said alteration being indicative of a predisposition to obesity.

The 5' flanking region of the *gad*2 gene is represented by nucleotides 1-2379 of SEQ ID N° 2, and the SNPs according to the invention are located at nucleotides 376 (presence of a T in predisposed patients, and a A in non-predisposed patients), 780 (presence of a A in predisposed patients, and a G in non-predisposed patients), and 2137 (presence of a G in predisposed patients, and a A in non-predisposed patients) of SEQ ID N° 2.

The invention relates more generally to the study of the 5' promoter region of the *gad*2 gene. Indeed, the inventors have demonstrated that the presence of the specific SNPs indicated in the present application leads to increased binding of nuclear factors to the 5' region of the *gad*2 gene, thus leading to an increase in transcription.

It is thus credible to speculate that other modifications in the promoter region of the *gad*2 gene, leading to a higher expression of the GAD2 protein will also lead to predisposition to obesity, due to increase in the GABA pool. Due to the orexigenic effect of GABA, this may alter feeding behavior.

Thus, in one embodiment, the invention relates to a method for diagnosing a predisposition for obesity in a human subject, wherein the level of an expression product of the *gad*2 gene in said sample is investigated.

The expression products according to the invention are meant to comprise RNA or protein, or what could be called "secondary" expression product, such as GABA. The latest is indeed not really an expression product of the *gad*2 gene, but increase in the production of GAD2 protein leads to increase in the GABA concentration.

Alteration of mRNA expression can be detected by any techniques known in the art. These include Northern blot analysis, PCR amplification and RNase protection

In one embodiment, mRNA of the sample is contacted with a *gad*2 gene probe under conditions suitable for hybridization of said probe to a RNA corresponding to said *gad*2 gene and hybridization of said probe is determined, and the level of signal after hybridization is compared with a standard (reference) signal (which is obtained from either a non-obese patient, or an obese patient).

The hybridization complex emits a signal, which may be due to the labeling of the probe, or of the mRNA. The different labels that may be used are well known to the person skilled in the art, and one can cite ³²P, ³³P, ³⁵S, ³H or ¹²⁵I. Non radioactive labels may be selected from ligants as biotin, avidin, streptavidin, dioxygenin, haptens, dyes, luminescent agents like radioluminescent, chemoluminescent, bioluminescent, fluorescent or phosphorescent agents.

In order to identify the SNPs mentioned in the present application, it is possible to contact a *gad*2 gene 5' region probe with genomic DNA isolated from said sample under conditions suitable for hybridization of said probe to said gene and hybridization of said probe is determined.

Said *gad*2 gene 5' region probe is either a "wild-type" DNA, (i.e. the searched SNP is not present on the probe, and hybridization occurs when no SNP according to the invention is present on the DNA in the sample) or a "mutant" one (i.e. carrying the searched SNP, and hybridization only occurs if the SNP is present on the DNA in the sample).

The person skilled in the art knows the techniques to determine the allele specific probes to use in this embodiment, their lengths, or the hybridization conditions. High stringency conditions are preferable in order to prevent false positives, for example under high stringency conditions of 0.2 x SSC and 0.1 % SDS at 55-65°C, or under conditions as described below.

The stringent hybridization conditions may be defined as described in Sambrook et al. ((1989) Molecular cloning : a laboratory manual. 2^{nd} Ed. Cold Spring Harbor Lab., Cold Spring Harbor, New York.), with the following conditions: 5 x or 6 x SCC, 50-65°C. Highly stringent conditions that can also be used for hybridization are defined with the following conditions: 6 X SSC, 60-65°C.

Hybridization ADN-ADN or ADN-ARN may be performed in two steps: (1) prehybridization at 42°C pendant 3 h in phosphate buffer (20 mM, pH 7.5) containing 5 or 6 x SSC (1 x SSC corresponding to a solution 0.15 M NaCl + 0.015 M sodium citrate), 50 % formamide, 7 % sodium dodecyl sulfate (SDS), 10 x Denhardt's, 5 % dextran sulfate et 1 % salmon sperm DNA; (2) hybridization during up to 20 at a temperature of 50-65°C, more preferably 60-65°C followed by different washes (about 20 minutes at in 2 x SSC + 2 % SDS, then 0.1 x SSC + 0.1 % SDS). The last wash is performed in 0.2 x SSC + 0.1 % SDS for about 30 minutes at about 50-65°C, and/or in 0.1 x SSC + 0.1 % SDS at the same temperature. These high stringency hybridization conditions may be adapted by a person skilled in the art. Indeed, the person skilled in the art is able to determine the best stringency conditions by varying the concentrations in SSC and SDS and the temperature of hybridization and washings.

In another embodiment, said expression product is the polypeptide encoded by the *gad*2 gene in said sample. In particular, said polypeptide may be detected by immunoblotting or immunocytochemistry.

Antibodies specific for GAD2 can be used to detect increased GAD2 expression. Immunological assays can be done in any convenient formats known in the art. These include Western blots, immunohistochemical assays and ELISA assays.

Thus, antibodies that react with the the GAD2 polypeptide, as well as reactive fragments of such antibodies, are also encompassed within the scope of the present invention. The antibodies may be polyclonal, monoclonal, recombinant, chimeric, single-chain and/or bispecific. In preferred embodiment, the antibody or fragment thereof will either be of human origin, or will be "humanized", i.e., prepared so as to prevent or minimize an immune reaction to the antibody when administered to a patient.

The antibody fragment may be any fragment that is reactive with the polypeptides of the present invention. the invention also encompasses the hybridomas generated by presenting the polypeptide according to the invention or a fragment thereof as an antigen to a selected mammal, followed by fusing cells (e.g., spleen cells) of the mammal with certain cancer cells to create immortalized cell lines by known techniques, such as the technique of Köhler et Milstein (1975 Nature 256, 495).

The antibodies according to the invention are, for example, chimeric antibodies, humanized antibodies, Fab ou F(ab')₂ fragments. They may be immunoconjugates or labeled antibodies, for detection purposes.

In another embodiment, the invention is performed by determining whether there is an alteration in the germline sequence of the *gad*2 gene 5' region in said sample by observing shifts in electrophoretic mobility of single-stranded DNA from said sample on denaturing or non-denaturing polyacrylamide gels. Said single-stranded nucleic acids may be obtained after amplification of the genomic DNA, using suitable primers, and denaturation (the gel and electrophoresis conditions are usually denaturing for such a purpose).

In another embodiment, the invention is performed by amplification of all or part of the *gad*2 gene 5' region from said sample, and determination of the sequence of said amplified DNA, for example by chain termination extenstion.

In another embodiment, allele specific oligonucleotide primers are employed to determine whether a specific *gad*2 mutant allele is present in said sample, the amplification only occurring in this case. This method is well known in the art.

In another embodiment, all or part of the *gad*2 gene 5' region from said sample is cloned to produce a cloned sequence and the sequence of said cloned sequence is determined. The cloning is performed in vectors known in the art, such as pUC or pBR vectors.

In general, the invention relates to a method for determining a predisposition to obesity, in particular morbid obesity, in a subject, from a sample of said subject, which comprises determining whether there is a mismatch between (1) the 5' region of the *gad*2 gene genomic DNA isolated from said sample, and (2) a nucleic acid probe complementary to human wild-type 5' region of the *gad*2 gene DNA, as represented by SEQ ID N° 2, when molecules (1) and (2) are hybridized to each other to form a duplex, and said mismatch being due to the presence of at least one of the 3 identified SNPs, present et nucleotides 376, 780 or 2137 of SEQ ID N° 2.

In another embodiment, the invention relates to a method wherein amplification of *gad*2 gene 5' flanking region sequences in said sample is carried out and hybridization of the amplified sequences to one or more nucleic acid probes issued from the wild-type *gad*2 gene 5' flanking region sequence (as represented in SEQ ID N° 2) or a mutant *gad*2 gene 5' flanking region sequence (in which at least one of the three SNPs located at nucleotides 376, 780 or 2137 of SEQ ID N° 2 is present), is determined.

The invention also relates to a a method which comprises determining *in situ* hybridization of the *gad*2 gene 5' flanking region in said sample with one or more nucleic acid probes issued from a wild-type or a mutant *gad*2 gene 5' flanking region sequence.

The invention opens up a new area in the treatment and/or prevention of obesity, in particular morbid obesity, especially for patient in families where genetic susceptibility is suspected.

Indeed, the presence of the SNPs in the 5' region of the *gad*2 gene, and the data showing increased binding of nuclear factors to said region when the polymorphisms identified in the frame of the invention are present, make it likely and credible that presence of these polymorphisms increase expression of *gad*2, probably through increased transcription. Furthermore, increased activity of *gad*2 gene leads to increase of the GABA pool, of the orexigenic effect of GABA, which may alter feeding behavior and contribute to the development of morbid obesity for the carriers of the identified mutations.

Thus, the invention also relates to a method for screening potential obesity drugs which comprises: combining (i) a compound suspected of being a obesity drug, (ii) a GAD2 polypeptide and determining the amount of binding of the GAD2 polypeptide to said compound. Indeed, inhibitors of the GAD2 enzyme will interfere with the formation of GABA and can be considered as useful drugs for treating obesity, alone or in association with other treatments.

The invention also relates to a method for screening potential obesity therapeutics which comprises: combining (i) a GAD2 binding partner, (ii) a GAD2 polypeptide and (iii) a compound suspected of being a obesity therapeutic and determining the amount of binding of the GAD2 polypeptide to its binding partner.

In a particular embodiment, said binding partner is L-glutamic acid.

The invention also relates to a method for screening potential obesity therapeutics which comprises: combining (i) a *gad*2 gene binding partner, (ii) a gad2 gene and (iii) a compound suspected of being a obesity therapeutic and determining the amount of binding of the *gad*2 gene to its binding partner. In this embodiment, the term "*gad*2 gene" must be understood as including the 5' flanking region in the *gad*2 locus, which comprises the polymorphisms of the invention, especially -243 A>G.

In particular, said *gad*2 gene binding partner is IK2 (Ikaros 2), the amino acid sequence of which is represented by SEQ ID N° 3.

The methods for assessing the binding of a compound to a nucleic acid or a protein are well known in the art, and are preferably performed *in vitro.* A method to achieve such a goal may be to link the nucleic acid or the protein to a solid support on which the compound to test is flown, and to check the recovery of the compound after passage on the support. By adjusting parameters, it is also possible to determine the binding affinity. The compounds may also be found by other methods including FRET, SPA... when the compounds and the nucleic acid or protein are labeled. The assay may also be performed on the cells containing the nucleic acid of the invention, for example on a vector according to the invention, and/or expressing the protein according to the invention. This also gives the information of the capacity of the compound to go through the membrane and penetrate within cells. These cells can be bacterial cells, or mammalian cells.

The method of the invention also allows the screening, detection and/or identification of compounds able to inhibit the biological activity of the GAD2 protein, and n particular the increase of the amount of produced GABA.

The present invention thus allows the detection, identification and/or screening of compounds that may be useful for the treatment of diseases where V(D)J recombination and/or DNA repair is involved. Nevertheless, the compounds identified by the method according to the invention, in order to be used in a therapeutic treatment, may need to be optimized, in order to have a superior activity and/or a lesser toxicity.

Indeed, the development of new drugs is often performed on the following basis:
- screening of compounds with the sought activity, on a relevant model, by an appropriate method,
- selection of the compounds that have the required properties from the first screening test (here, modulation of GAD2 or GABA production),
- determination of the structure (in particular the sequence (if possible the tertiary sequence) if they are peptides, proteins or nucleic acids, formula and backbone if they are chemical compounds) of the selected compounds,
- optimization of the selected compounds, by modification of the structure (for example, by changing the stereochemical conformation (for example passage of the amino acids in a peptide from L to D), addition of substituants on the peptidic or chemical backbones, in particular by grafting groups or radicals on the backbone, modification of the peptides (se in particular Gante "Peptidomimetics", in Angewandte Chemie-Intemational Edition Engl. 1994, 33. 1699-1720),
- testing and screening of the "optimized" compounds on appropriate models that are often models nearer to the studied pathology. At this stage, one would often use animal models, in particular rodents (rats or mice) or dogs or non-human primates, that are good models of obesity, and to look for the phenotypic changes in said models after administration of the compound.

The invention also relates to a pharmaceutical composition comprising a pharmaceutically acceptable excipient and a compound as defined in claim 10 and to the use of a compound identified by a method according to the invention as defined in Claims 11 or 12 for the manufacture of a drug intended to treat and/or prevent obesity, especially morbid obesity.

The invention also relates to a method for the therapy of obesity, in particular morbid obesity, comprising administration of a compound or a pharmaceutical composition of the invention.

In order to decrease the activity of the *gad*2 gene and protein, it is possible to use antisense molecules, in particular complementary to the mRNA of *gad*2, in a pharmaceutical composition for treating obesity. The invention relates to a method for the therapy of obesity, comprising administration of anti-*gad*2 antisense molecule or any means to decrease the amount of GAD2 protein. The person skilled in the art is aware of the means to design antisense molecules, and of the modifications that can be brought to the backbones of the molecules (phosphorothioates, methylphosphonates...).

Antisense polynucleotide sequences are useful in preventing or diminishing the expression of the *gad*2 gene, as will be appreciated by those skilled in the art. For example, polynucleotide vectors containing all or a portion of the *gad*2 gene (or other sequences from the *gad*2 locus, especially the 5' flanking region) may be placed under the control of a promoter in an antisense orientation and introduced into a cell. Expression of such an antisense construct within a cell will interfere with *gad*2 transcription and/or translation.

Alternatively, a "sense" strategy may be foreseen, where a nucleic acid oligonucleotide or probe comprising part of the 5' region of the *gad2* gene (especially comprising the -243 A>G variant at nucleotide 2137 of SEQ ID N° 2) is introduced within the cells, in order to be competitor for binding of the nuclear factors activating transcription. The size of the fragment of the 5' flanking region of the *gad*2 gene that can be used in the sense strategy is preferably about 50-150 bases.

The invention also relates to the use of animal models for studying obesity, where expression of the *gad*2 gene is increased. In particular, expression of this gene can be increased only conditionally, using promoters that are either site- or time-specific, or inducible promoters. It is thus possible to increase *gad*2 expression only in the pancreas of the animals according to the invention, or in the brain. The methods to obtain animals according to the invention are known by the persons skilled in the art, and are useful in particular for testing some drugs that can be identified according to the methods of the invention.

The insertion of a construct in the genome of an animal to obtain a transgenic animal may be performed by methods well known by the artisan in the art, and can be either random or targeted. In a few words, the person skilled in the art will construct a vector containing the sequence to insert within the genome, with an appropriate promoter, and a selection marker (for example the gene coding for the protein that gives resistance to neomycine), and may have it enter in the Embryonic Stem (ES) cells of an animal. The cells are then selected with the selection marker, and incorporated into an embryo, for example by microinjection into a blastocyst, that can be harvested by perfusing the uterus of pregnant females. Reimplantation of the embryo and selection of the transformed animals, followed by potential back-crossing makes it possible to obtain such transgenic animal. To obtain a "cleaner" animal, the selection marker gene may be excised by use of a site-specific recombinase, if flanked by the correct sequences.

According to one embodiment, the transgenic non-human mammal has integrated into its genome the nucleic acid sequence of the *gad*2 gene or the coding sequence thereof, operatively linked to regulatory elements, wherein expression of said coding sequence increases the level of the GAD2 protein and/or the GABA pool in said mammal relative to a non-transgenic mammal of the same species. Transgenic mice having an increased expression of gad2 are described in WO-A- 98 /37224 or Geng et al, PNAS vol. 95, 1995, p.10055 - 10060.

The *gad*2 sequence foreseen for the preceding embodiment may be a human *gad*2 gene sequence introduced within the genome of the animal of the invention, or the endogenous *gad*2 gene sequence the promoter of which has been modified in order to induce overexpression. For example, the *gad*2 coding sequence for mouse is knoan and may be found in GenBank under number NM_008078. Other endogenous *gad*2 genes in other animals may be identified using the homologies between sequences.

Alternatively, a transgenic non-human mammal whose genome comprises a disruption of the endogenous *gad*2 gene, is used as an animal model for testing links bewteen GAD2 and obesity. In particular, said disruption comprises the insertion of a selectable marker sequence, and said disruption results in said non-human mammal exhibiting a defect in GABA level as compared to a wild-type non-human mammal.

In particular, said disruption is a homozygous disruption, and said homozygous disruption results in a null mutation of the endogenous gene encoding GAD2.

US 6,087,555 describes one way of obtaining a knock-out mouse, and the general teaching of this patent is incorporated herein by reference (column 5, line 54 to column 10 line 13). In this patent, it is described an OPG knock-out mouse, but the same method applies to a GAD2 knock-out mouse. The person skilled in the art will also find information in Hogan et al. (Manipulating the Mouse Embryo: a Laboratory Manual, Cold Spring Harbor Laboratory Press, Cold Spring Harbor, NY; 1986).

The mammal of the invention is preferably a rodent, especially a rat or a mouse. GAD65 (gad2) knockout mice are described in Fagiolini and Hensch, Nature vol. 404, 2000, p.183 - 186.

### DESCRIPTION OF THE FIGURES

Figure 1: schematic representation of the *gad*2 gene, and of the frequency of the SNPs identified in the frame of the invention.
Figure 2: gel shift assay, testing the binding of nuclear protein in the 5' region of the *gad2* gene, depending of the nature of the nucleotide at position -243. mt = mutant (G); wt = wild type (A).
Figure 3: Effect of -243A>G *gad*2 variant on transcriptional activity in βTC3 cells. Relative Luciferase Units are expressed as means ± S.D.

### EXAMPLES

### Example 1: association between obesity and SNPs

The Gad2 gene, encodes an isoform of glutamic acid decarboxylase (GAD65), that catalyses the formation of gamma aminobutyric acid (GABA) from L glutamic acid, and is expressed in both brain and pancreatic islets cells. GABA is one of the most powerful inhibitory neurotransmitters of the central nervous system (CNS). GABA modulate the gastrin's and somatostatin's secretion and may stimulate the glucose intake in several tissues (Erdo and Wolff, 1990 J Neurochem.;54(2):363-72.).

Three SNPs (-243 A>G variant (5'UTRI); -1.6kb G>A (SNP 668); -2004 A>T (SNP 669)), located in the 5' flanking region of the *gad*2 gene revealed positive association with obesity in morbidly obese subjects.

The DNA fragments bearing these polymorphisms can be amplified with the following sets of primers,

Polymorphisms of the *gad*2 gene were genotyped by direct sequencing or by the LightCycler™ technology (Roche, Manheim, Germany). The LightCycler assay is based on hybridization probes labelled with fluorescent dyes that allow fluorescence resonance energy transfer (Blomeke et al., 1999, *Anal Biochem* , **275,** 93-7). SNP genotyping was carried out using melting curves analysis. PCR are performed in 9700 apparatus (Applied Biosytems, Foster city,USA) before analysis in lightCycler. Sequences of primers used for PCR and for the LightCycler assays are described in the following table.

| SNPs | PCR primers | Primers for Light cycler assay |
|---|---|---|
| -243 A>G | F: 5'cctcaaatgctctggggctc3' R: 5'ggtgtcacgcaggaacagaa3' | Red640-gtctcttttaaagctccccggct cgggctccgaggacccttaggtagtccc-F |
| -1600 G>A | F 5'ctgaggcgtattaggag R: 5'ctcctaatacgcctcag | Red640-ggaaagcagccgcctc tggaaatgacaggcgctctggccaggcgcg-F |
| -2004 A>T | F: 5'tgttttcaaccactcatccat R ; 5'agggacagttatgctcg | Red640-acagcctggtacagactt tgagttttcgaccacccgggctc-F |

| | | |
|---|---|---|
| (F represents the fluorescein tag). | | |

PCR amplifications were performed in a final reaction volume of 20 □1 PCR buffer, containing 50 ng human genomic DNA, 10 pmol of each primer, 2,5 mmol/l MgCl2, 2,5 mmol/l of dNTP, and 0,2 U of Taq Gold polymerase. The thermal cycling conditions for the PCR product were : 1 cycle 95°C 12 min ; 35 cycles, 95°C 15 sec, 55°C 15 sec, 72 °C 30 sec ;1 cycle 72°C 10 min ; 1 cycle 15°C 15 min.

In addition, in order to study the restrained eating and "latent obesity", cognitive restraint of eating, disinhibition and hunger were analyzed according the "Three factor eating questionnaire or TFEQ" (Stunkard AJ and Messick S, 1985 J Psychosom Res; 29(1):71-83.).

Association studies were performed in 369 morbidly unrelated obese patients (mean BMI: 47.3 ± 7.4 kg/m2; mean age: 46 ± 12 years; women/men, 294/75).

A set of 381 unrelated non obese non diabetic subjects (mean BMI: 22.8 ± 2.44 kg/m²; mean age: 58.3 ± 14.1 years; women/men, 228/152) was used as a control group. Table 1 shows genotype distribution and allele frequencies of the 3 SNPs (-243 A>G variant (5'UTRI); -1.6kb G>A (SNP 668); -2004 A>T (SNP 669)).

**Table .Genotype distribution and allele frequencies of 242 A>G, 1.6kb G>A and 1.7kb A>T SNPs**

| Codominant Model | | Morbidly obeses BMI> 40 | | Controls | | *p-value* |
|---|---|---|---|---|---|---|
| SNP | Genotype | **number of genotypes** | Allele Freq. | **numberof genotypes** | Allele Freq. | |
| 5'UTR: | AA | 233 | A:79.9% | 270 | A:84.5% | p=0.05 |
| -243 A>G | AG | 117 | G:2o.1°io | 92 | 6:15.5% | |
| | GG | 15 | | 12 | | |
| SNP668: | GG | 229 | 6:802% | 255 | 6:84.8% | p=0.06 |
| -1.6kb G>A | GA | 1 13 | A:19.8% | g7 | A:152% | |
| | AA | 14 | | 10 | | |
| SNP669: | AA | 221 | A:80.4% | 254 | A:83.8% | p=0.19 |
| -2004 A>T | AT | 105 | T:19.6% | g9 | T:16.2% | |
| | AA | 14 | | 13 | | |
| | | | | | | |

| Dominant Model | | **Morbidly obeses** BMI>40 | | Controls | | *p-value* |
|---|---|---|---|---|---|---|
| SNP | Genotype | **number of genotypes** | Allele Freq. | **number of genotypes** | A]lele Freq. | |
| 5'UTR: | AA | 233 | A:79.9% | 270 | A:84.5% | p=0.004 |
| -243 A>G | AG/GG | 132 | 6:20.1% | 104 | 6:15.5% | |
| SNP668: | GG | 229 | 6:80.2% | 255 | 6:84.8% | p=0.02 |
| -1.6kb G>A | GA/AA | 127 | A:19.8% | 97 | A:15.2% | |
| SNP669: | AA | 221 | A:80.4% | 254 | A:83.8% | p=0.07 |
| -2004 A>T | AT/AA | 119 | T:19.6% | 102 | T:16.2% | |

Under codominant and dominant model, the -243 A>G and the -1.6kb G>A SNP were associated with morbid obesity (respective p values of 0.004 and 0.02 under a dominant model). A trend toward association was observed for the -2004 A>T variant.

The relative risk of these 3 SNPs was estimated: O.R = 1.55 (95% CI [1.14 2.12]) (UTR5I; -243); O.R = 1.45 (95% CI [1.06 2]) (SNP668; -1.6kb); O.R = 1.34 (95% CI [0.97 1.84]) (SNP669; -2004).

In order to replicate these results, the 3 SNPs were genotyped in another set of 316 unrelated morbidly obese subjects. Similar result was obtained for the -243 A>G (under dominant model, p = 0.009) and a trend toward was obtained for the -1.6kb G>A SNP (under dominant model, p = 0.06).

Analysis of variance for obesity related phenotypes was performed (BMI, leptin, percent fat mass, insulinemia...) and significant association between the 3 SNPs and the three factors of eating behavior was found.

To test the human eating behavior, the subjects filled in the TFEQ (Three Factor Eating Questionnaire) questionnaire established by Stunkard et al. (*op. cit.).* The analysis of variance for three stable factors measured by TFEQ: cognitive restraint of eating (Qres), disinhibition (Qdis) and hunger (Qhun) is show in table 2. Linkage disequilibrium between the 3 SNPs was significant (χ² = 174,p<10⁻¹⁰).

Linkage disequilibrium between each SNP was estimated by Estimation Haplotype (EH) software. D' = 0.84 (SNPs -243 A>G and -1.6 kb G>T), D' = 0.84 (SNPs -243 A>G and -2004 kb G>A), D' = 0.87 (SNPs -1.6 kb G>T and -2004 kb G>A).

Table 2 shows the results obtained for the -243 A>G (5'UTR) variant. Similar results were obtained for the -1.6kb G>A (SNP 668) and the -2004 A>T (SNP 669) variants.

**Table 2 Analyses of variance with fond intake behavior parameters.**

| | | | | |
|---|---|---|---|---|
| -243 A>G (5'UTRI) | | | | |
| | AA | AG | GG | P |
| N | 233 | 117 | 15 | |
| Age | 46.02±0.78 | 47.82±1.1 | 39.9±3.1 | 0.04 |
| Age-Pmax | 43.5±0.79 | 44.3±1.1 | 35.76±3.1 | 0.03 |
| Qres | 9.53±0.31 | 9±0.45 | 6.53±1.28 | 0.06 |
| Qdis | 8.38±0.24 | 8.71±0.34 | 10.9±0.96 | 0.03 |
| Qhun | 5.29±0.24 | 6.49±0.35 | 7.95±0.95 | 0.001 |

Using a codominant model, it was found that the GG carriers presented a lower restriction score than heterozygous and wild type subjects (pvalue = 0.06). They also presented a higher disinhibition (pvalue = 0.03) and a higher hunger scores (pvalue = 0.001) than heterozygous and wild type subjects. Furthermore, it was observed that GG carriers are younger (pvalue = 0.04) and seem to reach a maximal weight in early ages than AG and AA carriers (pvalue = 0.03). These data suggest that alterations of feeding behavior by *gad*2 variants can contribute to the development of younger onset of morbid obesity.

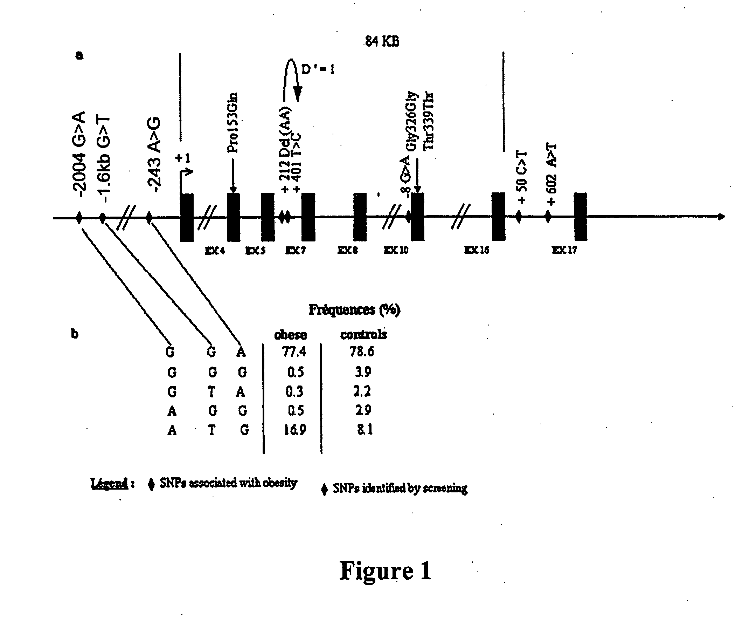

Haplotypes studies were performed with the -243 A>G, -1.6kb G>A and the -2004 A>T variants (EHplus software). A significant haplotype frequency difference between obese and non obese subjects was observed (p <10⁻¹⁰). The ATG haplotype was shown to be more frequent in obese (16.9%) compared to controls (8%) (Figure 1).

### Example 2: Functional study of a promoter variant of gad 2 gene

To search for potential binding sites, the sequence of the 5' flanking region of the *gad*2 gene was submitted to the transfac server: http://transfac.gbf.de/cgibin/mat.

*In silico* analyses showed that the -243 A>G (located at nucleotide 2137 of SEQ ID N° 2) and the -1.6kb G >A (located at nucleotide 780 of SEQ ID N° 2) were located near a predicted Ikaros 2 (IK2) response element site. IK2 is a transcription factor that is highly expressed in human lymphocytes, and its amino acid sequence is represented by SEQ ID N° 3.

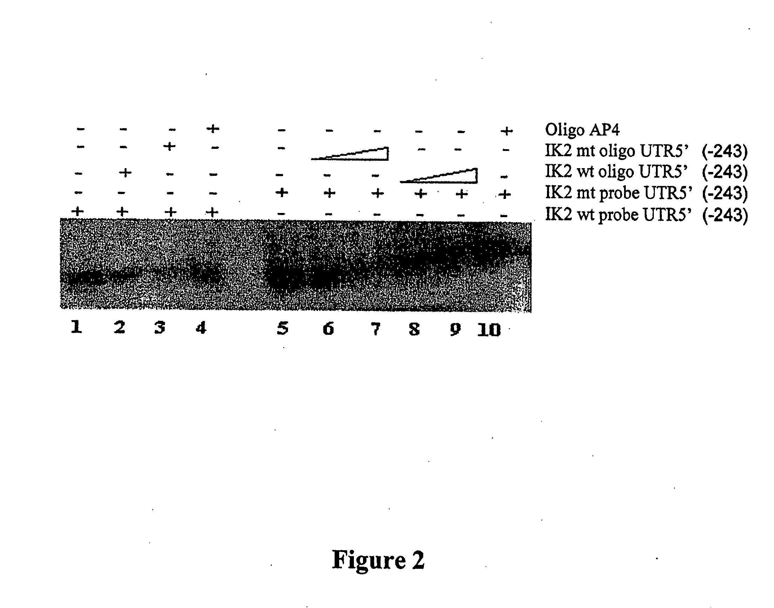

In order to test if the -243 A>G variant alters nuclear protein binding to the DNA, a gel shift assay using primers with sequences of the wild type and mutated site and a nuclear extract from MIN6 cells was performed (Figure 2).

Results of the gel shift assay showed that there is *in vitro* binding of nuclear proteins to the IK2 responsive element site. Apparently, the nuclear proteins have a higher affinity to the response element G variant (lire 6 versus 1). Similar result were obtained with competition of the A and G allele (lire 2 vs 3; 6 vs 8; 7 vs 9).

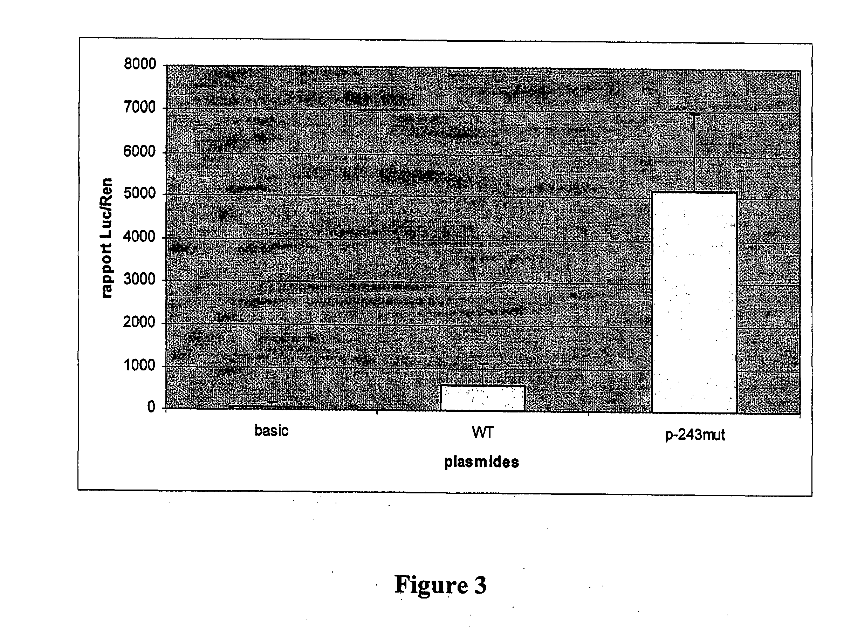

To investigate if the allelic variant at position -243 influences GAD2 transcriptional level, transient cotransfections using firefly luciferase reporter construct were performed to measure proximal promoter activity of wild type and variant promoters in βTC3 cells (murine insulinoma cell line). Renilla vector was used to normalize transfection efficiency. The transcriptional activity of -243A>G mutant construct was 8.61 times higher compared to the wild type construct (n=8, p<0.0001) (Figure 3)

The observed increased transcriptional activity induced by the G allele of the -243 A>G is in concordance with the genetic results
- the association of the -243 A>G variant with obesity (p=0.004, O.R =1.55 (95% CI [1.14-2.12])
- The GG bearers have a lower food restriction score (p = 0.06), a higher disinhibition score (p = 0.03) and a higher hunger score parameter (p = 0.001).

These results indicate that the binding of nuclear proteins (potentially the IK2 transcription factor), in presence of variant allele, was increased and may transactivate the Gad2 gene, and thus the GABA pool. Therefore, orexigenic effect of GABA might be increased and might alter feeding behaviour and contribute to the development of morbid obesity for G carriers of the -243 A>G variant.

The result may be generalized to the other SNPs, that are also close to IK2 response element.

The evidence presented in this application indicates involvement of the GABA pathway in obesity in human. This is an important step towards a better understanding of the molecular mechanisms leading to common forms of obesity. It seems clear that the GABAergic neurons are involved in the integration of signals modulating food intake. The identification of SNPs in the 5' region of the *gad*2 gene and the demonstration that their presence modulates the expression of a key enzyme (GAD2), which in turn will modify the GABA pool opens new perspective for drugs against obesity.

## Claims

1. A method for diagnosing a predisposition for obesity, and in particular morbid obesity, in a human subject which comprises determining whether there is a germline alteration in the sequence of the 5' flanking region of the *gad*2 gene, the coding sequence of which is represented by SEQ ID N° 1, wherein said alteration is the presence of at least one of the following mutations: -243 A>G at nucleotide 2137 of SEQ ID N° 2, -1.6kb G>A at nucleotide 780 of SEQ ID N° 2, -2004 A>T at nucleotide 376 of SEQ ID N° 2, said alteration being indicative of a predisposition to obesity.

2. The method of claim 1, wherein said obesity is morbid obesity.

3. A method for diagnosing a predisposition for obesity in a human subject, from a sample from said subject, wherein the level of an expression product of the *gad*2 gene in said sample is investigated.

4. The method of claim 3, wherein said expression product is RNA or protein, or GABA.

5. A method for screening potential obesity drugs which comprises: combining (i) a compound suspected of being an obesity drug, (ii) a GAD2 polypeptide and determining the amount of binding of the GAD2 polypeptide to said compound.

6. A method for screening potential obesity therapeutics which comprises: combining (i) a GAD2 binding partner, (ii) a GAD2 polypeptide and (iii) a compound suspected of being a obesity therapeutic and determining the amount of binding of the GAD2 polypeptide to its binding partner.

7. The method of claim 6, wherein said GAD2 binding partner is L-glutamic acid.

8. A method for screening potential obesity therapeutics which comprises: combining (i) a *gad*2 gene binding partner, (ii) a *gad*2 gene and (iii) a compound suspected of being a obesity therapeutic and determining the amount of binding of the *gad2* gene to its binding partner.

9. The method of claim 8, wherein said *gad2* gene binding partner is IK2 (Ikaros 2).

10. A pharmaceutical composition comprising a sense molecule comprising a fragment of the 5' flanking region of the *gad*2 gene, especially comprising the -243 A>G variant (at nucleotide 2137 of SEQ ID N° 2) within said region and a pharmaceutically acceptable excipient.

11. Use of an antisense molecule complementary to the *gad*2 mRNA for the preparation of a drug intended for treatment of obesity, in particular morbid obesity.

12. Use of a sense molecule comprising a fragment of the 5' flanking region of the *gad*2 gene, especially comprising the -243 A>G variant (at nucleotide 2137 of SEQ ID N° 2), within said region, for the manufacture of a drug intended for the treatment of obesity.

13. Use of a transgenic non-human mammal having integrated into its genome the nucleic acid sequence of *gad*2, or coding sequence thereof, operatively linked to regulatory elements, wherein expression of said sequence increases the level of the GAD2 protein and/or the GABA pool in said mammal relative to a non-transgenic mammal of the same species, as a model for studying obesity, or for testing potential anti-obesity drugs.

14. Use of a transgenic non-human mammal whose genome comprises a disruption of the endogenous *gad*2 gene, wherein said disruption comprises the insertion of a selectable marker sequence, and wherein said disruption results in said non-human mammal exhibiting a defect in GABA level as compared to a wild-type non-human mammal, as a model for studying obesity, or for testing potential anti-obesity drugs.

15. Use according to claim 13 or 14, wherein the mammal is a mouse.

## Patentansprüche

1. Verfahren zum Diagnostizieren einer Prädisposition für Fettleibigkeit und insbesondere krankhafte Fettleibigkeit bei einem menschlichen Subjekt, welches das Bestimmen umfasst, ob eine Keimlinienänderung in der Sequenz der 5' flankierenden Region des *gad*2-Gens vorliegt, dessen codierende Sequenz durch SEQ ID Nr. 1 dargestellt wird, wobei die Änderung die Anwesenheit mindestens einer der folgenden Mutationen ist: -243 A>G am Nucleotid 2137 von SEQ ID Nr. 2, -1,6 kb G>A am Nucleotid 780 von SEQ ID Nr. 2, -2004 A>T am Nucleotid 376 von SEQ ID Nr. 2, wobei die Änderung eine Prädisposition für Fettleibigkeit anzeigt.

2. Verfahren nach Anspruch 1, in dem die Fettleibigkeit krankhafte Fettleibigkeit ist.

3. Verfahren zum Diagnostizieren einer Prädisposition für Fettleibigkeit bei einem menschlichen Subjekt aus einer Probe des Subjekts, in dem die Konzentration eines Expressionsprodukts des *gad*2-Gens in der Probe untersucht wird.

4. Verfahren nach Anspruch 3, in dem das Expressionsprodukt RNA oder Protein oder GABA ist.

5. Verfahren zur Durchmusterung potentieller Fettleibigkeitsarzneistoffe, welches umfasst: Vereinigen (i) einer Verbindung, von der vermutet wird, dass sie ein Fettleibigkeitsarzneistoff ist, (ii) eines GAD2-Polypeptids und Bestimmen der Menge der Bindung des GAD2-Polypeptids an die Verbindung.

6. Verfahren zur Durchmusterung potentieller Fettleibigkeitstherapeutika, welches umfasst: Vereinigen (i) eines GAD2-Bindungspartners, (ii) eines GAD2-Polypeptids und (iii) einer Verbindung, von der vermutet wird, dass sie ein Fettleibigkeitstherapeutikum ist, und Bestimmen der Menge an Bindung des GAD2-Polypeptids an seinen Bindungspartner.

7. Verfahren nach Anspruch 6, in dem der GAD2-Bindungspartner L-Glutaminsäure ist.

8. Verfahren zum Durchmustern von potentiellen Fettleibigkeitstherapeutika, welches umfasst: Vereinigen (i) eines *gad*2-Bindungspartners, (ii) eines gad2-Gens und (iii) einer Verbindung, von der vermutet wird, dass sie ein Fettleibigkeitstherapeutikum ist, und Bestimmen der Menge an Bindung des gad2-Gens an seinen Bindungspartner.

9. Verfahren nach Anspruch 8, in dem der *gad*2-Gen-Bindungspartner IK2 (Ikaros 2) ist.

10. Pharmazeutische Zusammensetzung, umfassend ein Sense-Molekül, das ein Fragment der 5' flankierenden Region des *gad*2-Gens umfasst, die insbesondere die -243 A>G Variante (am Nucleotid 2137 der SEQ ID Nr. 2) innerhalb dieser Region umfasst, und eine pharmazeutisch annehmbaren Träger.

11. Verwendung eines Antisense-Moleküls, das komplementär zu der *gad*2-mRNA ist, für die Herstellung eines Medikaments zur Behandlung von Fettleibigkeit, insbesondere krankhafter Fettleibigkeit.

12. Verwendung eines Sense-Moleküls, das ein Fragment der 5' flankierenden Region des *gad*2-Gens umfasst, die insbesondere die -243 A>G Variante (am Nucleotid 2137 der SEQ ID Nr. 2) innerhalb der Region umfasst, für die Herstellung eines Arzneimittels, das zur Behandlung von Fettleibigkeit bestimmt ist.

13. Verwendung eines transgenen nicht-menschlichen Säugetiers, das in seinem Genom integriert die Nucleinsäuresequenz von *gad*2 oder die codierende Sequenz davon operativ mit Regulationselementen verknüpft aufweist, wobei die Expression der Sequenz die Konzentration des GAD2-Proteins und/oder den GABA-Pool in dem Säugetier relativ zu einem nichttransgenen Tier derselben Art erhöht, als Modell zur Untersuchung von Fettleibigkeit oder zum Testen von potentiellen Antifettleibigkeitsarzneistoffen.

14. Verwendung eines transgenen nicht-menschlichen Säugetiers, dessen Genom eine Zerstörung des endogenen *gad*2-Gens umfasst, wobei die Zerstörung die Insertion einer selektierbaren Markersequenz umfasst und wobei die Zerstörung zur Folge hat, dass das nicht-menschliche Säugetier einen Mangel der GABA-Konzentration im Vergleich zu eine nicht-menschlichen Wildtyp-Säugetier zeigt, als Modell zur Untersuchung von Fettleibigkeit und zum Testen von potentiellen Antifettleibigkeitsarzneistoffen.

15. Verwendung nach Anspruch 13 oder 14, wobei das Säugetier eine Maus ist.

## Revendications

1. Procédé pour diagnostiquer une prédisposition à l'obésité, et en particulier à l'obésité pathologique, chez un sujet humain, comprenant le fait de déterminer s'il existe une altération germinale dans la séquence de la région adjacente en 5' du gène *gad2*, dont la séquence codante est représentée par SEQ ID n° 1, ladite altération étant la présence d'au moins une des mutations suivantes : -243 A>G au niveau du nucléotide 2137 de SEQ ID n° 2, -1,6 kb G>A au niveau du nucléotide 780 de SEQ ID n° 2, -2004 A>T au niveau du nucléotide 376 de SEQ ID n° 2, ladite altération étant indicatrice d'une prédisposition à l'obésité.

2. Procédé selon la revendication 1, dans lequel ladite obésité est une obésité pathologique.

3. Procédé pour diagnostiquer une prédisposition à l'obésité chez un sujet humain, à partir d'un échantillon provenant dudit sujet, dans lequel on examine le taux d'un produit d'expression du gène *gad2* dans ledit échantillon.

4. Procédé selon la revendication 3, dans lequel ledit produit d'expression est de l'ARN ou une protéine, ou GABA.

5. Procédé pour le criblage de principes actifs potentiels contre l'obésité, comprenant la réunion (i) d'un composé suspecté d'être un principe actif contre l'obésité, (ii) d'un polypeptide GAD2, et la détermination de la quantité de liaison du polypeptide GAD2 audit composé.

6. Procédé pour le criblage d'agents thérapeutiques potentiels contre l'obésité, comprenant la réunion (i) d'un partenaire de liaison à GAD2, (ii) d'un polypeptide GAD2 et (iii) d'un composé suspecté d'être un agent thérapeutique contre l'obésité et la détermination de la quantité de liaison du polypeptide GAD2 à son partenaire de liaison.

7. Procédé selon la revendication 6, dans lequel ledit partenaire de liaison est l'acide L-glutamique.

8. Procédé pour le criblage d'agents thérapeutiques potentiels contre l'obésité, comprenant la réunion (i) d'un partenaire de liaison au gène *gad2*, (ii) d'un gène *gad*2 et (iii) d'un composé suspecté d'être un agent thérapeutique contre l'obésité et la détermination de la quantité de liaison du gène *gad*2 à son partenaire de liaison.

9. Procédé selon la revendication 8, dans lequel ledit partenaire de liaison au gène *gad*2 est IK2 (Ikaros 2).

10. Composition pharmaceutique comprenant une molécule sens comprenant un fragment de la région adjacente en 5' du gène *gad2*, en particulier comprenant le variant -243 A>G (au niveau du nucléotide 2137 de SEQ ID n° 2) dans ladite région, et un véhicule pharmaceutiquement acceptable.

11. Utilisation d'une molécule antisens complémentaire de l'ARNm de *gad2*, pour la fabrication d'un médicament destiné au traitement de l'obésité, en particulier de l'obésité pathologique.

12. Utilisation d'une molécule antisens comprenant un fragment de la région adjacente en 5' du gène *gad2*, en particulier comprenant le variant -243 A>G (au niveau du nucléotide 2137 de SEQ ID n° 2) dans ladite région, pour la fabrication d'un médicament destiné au traitement de l'obésité.

13. Utilisation d'un mammifère non-humain transgénique comportant intégrée dans son génome la séquence nucléotidique de *gad2*, ou des séquences codantes de celle-ci, liée de façon opérationnelle à des éléments régulateurs, l'expression de ladite séquence augmentant le taux de la protéine GAD2 et/ou du pool GABA chez ledit mammifère, par rapport à un mammifère non-transgénique de la même espèce, en tant que modèle pour la réduction de l'obésité ou pour tester de potentiels principes actifs anti-obésité.

14. Utilisation d'un mammifère non-humain transgénique dont le génome comprend une destruction du gène *gad2* endogène, dans laquelle ladite destruction comprend l'insertion d'une séquence de marqueur sélectionnable, et dans laquelle ladite destruction a pour résultat que ledit mammifère non-humain présente une anomalie au niveau GABA, par comparaison avec un mammifère non-humain de type sauvage, en tant que modèle pour l'étude de l'obésité ou pour tester de potentiels principes actifs anti-obésité.

15. utilisation selon la revendication 13 ou 14, dans laquelle le mammifère est une souris.
